# EUROPEAN PATENT APPLICATION

(11) **EP 4 481 029 A1**
(43) Date of publication of application: **25.12.2024**
(21) Application number: 23306001.1
(22) Date of filing: 22.06.2023
(51) Int. Cl.: C12M 1/00, C12M 1/36, C12M 1/32, C12M 3/06, C12M 1/04, C12M 1/34

(54) **METHOD FOR CONTROLLING FLUID DISPLACEMENT AND GAS(ES) COMPOSITION AND/OR PH IN A CELL CULTURE DEVICE AND CORRESPONDING CELL CULTURE SYSTEM**

(71) Applicant: Cherry Biotech SAS, 93100 Montreuil (FR)
(72) Inventor: HOMS CORBERA, Antoni de Padua, 17220 Sant Feliu de Guixols (ES)
(74) Representative: Vidon Brevets & Stratégie

(57) **Abstract**

The invention concerns a method for controlling fluid displacement and dissolved gas(es) composition and/or pH of a volume of liquid cell culture medium contained in at least one closed chamber. The method comprises:
- an injection of a gas, or gas mixture by at least one gas controller,
- an injection of a liquid cell culture medium by at least one liquid controller into said at least one closed chamber,
- a production of a diffusion and pressure driven exchange of gas, or gas mixtures, between said injected liquid cell culture medium and said injected gas, or gas mixture through at least one gas-permeable membrane,
- a collection of measurements of a dissolved gas(es) concentration and/or pH of said liquid cell culture medium by at least one dissolved gas(es) concentration and/or pH sensor,
- a determination of an injection flow rate and/or pressure of said gas, or gas mixture, and/or gas mixture composition, and/or an injection flow rate and/or pressure of said liquid cell culture medium based on said measured dissolved gas(es) concentration and/or pH of said liquid cell culture medium, fluid flow rate to be less or equal to 50ml/min, local pressures to be less or equal to 100 bar and a predetermined dissolved gas, or gas mixture, composition and/or pH of the liquid cell culture medium,
- an adjustment of an injection flow rate and/or pressure of said gas, or gas mixture by said at least one gas controller and/or an injection flow rate and/or pressure of said liquid cell culture medium by said at least one liquid controller based on said determination to obtain said predetermined dissolved gas, or gas mixture composition and/or pH of the liquid cell culture medium.

## Description

### Technical field

The invention relates to the field of *in vitro* culture of cells, tissues or organoids in culture plates, in particular so-called multi-well plates, or in fluidic devices.

In particular, the invention relates to the control of the environmental conditions of *in vitro* culture within a confined space such as cell culture plates, or in fluidic devices.

More particularly, the invention relates to a method for controlled dissolved gas(es) composition and/or pH of a flowing liquid cell culture medium and a cell culture system configured to implement the method for *in vitro* cell culture.

### Previous technique

*In vitro* cultures of cells, or of cellular assembly such as tissue culture, of spheroids or even more recently of organoids, i.e. of three-dimensional multicellular structures which reproduce *in vitro* the micro-anatomy or physiology of an organ, are today increasingly used as a tool for the toxicological evaluation of substances, in particular pharmacological or cosmetic, or biotherapies, such as those based on immune cells.

These *in vitro* models are used, particularly in pharmaceutical research, because they represent a serious alternative to *in vivo* models, i.e. to animal experimentation, against which both economic and ethical pressures appear at the international level.

Thus, to be able to carry out controlled biological experiments using these *in vitro* models and to analyze therapeutic compounds, cosmetics or potentially dangerous substances, it is essential to automatize the maintenance of cell culture parameters and the induction of selective environmental changes in confined cellular culture environments.

Microfluidic cell culture, understood as the cultivation of cells in chambers, or reservoirs, connected and fed by microchannels characterized by fluidic volumes between 1pL and 100mL, is an important technology for drug screening, tissue culture, detection of toxicity and biological research. It improves cell culture conditions, permits multiple cell types to communicate, as well as better control of chemical and physical mechanisms such as diffusion and shear-stress. Moreover, it provides better quality of experimental data, it economizes reagents consumption and it reduces costs.

International Patent Application WO2009126524, WO2016186503 and WO2021206551A1 describe examples of three-dimensional microfluidic devices for formation and study of three-dimensional biological systems capable of replicating critical biological functions.

On the other hand, the current standard tool for analytical research and clinical diagnostic tests used in the laboratory is the multi-well culture plate. These multi-well plates have been used for many years for cell and tissue culture.

For the protection of cell cultures against contamination by foreign germs or unwanted particles, the multi-well plates are closed with a matching lid. Depending on the cell type or the tissues growing in the wells, it is necessary to regularly change the cell culture medium, in order to renew the nutrients and/or the treatment applied to the cells (Paul J.: Cell and tissue culture 5th ed. Livingston, Edinburgh, 1975). Limitations of current use of these devices include the long-term culturing, i.e longer than 7 days. Indeed, using multi-well culture plate involves continuous exchange and enrichment of the cell culture medium. In addition, multi-well culture plate lacks of accurate control of the microenvironments near the cells and generation of controlled physical forces, such as shear stress, that can be critical to maintain the right phenotype of some cells populations and tissues as described by De Belly, Paluch and Chalut in Nature Reviews Molecular Cell Biology 23: 465-480 (2022).

Patent application WO 2009089189 (MILLIPORE CORPORATION) describes a microfluidic cell culture system comprising, among other things, a microfluidic chamber making it possible to reduce the cell culture conditions. The pressurized movement of liquids to the microfluidic cell culture chamber allows high precision to be achieved, even for very small volumes. Fast laminar flow switching between five flow inlet solutions, along with perfusion barriers enable continuous mass transport without shear stress on the cells. In particular, this system allows live cell analysis experiments to be carried out for more than three days, but not more than five days on the stage of any standard inverted microscope. This system also has gaseous environment and temperature controls.

However, this system is not without drawbacks. Indeed, the high complexity of this system leads to a high failure rate of the integrated device. The gas enrichment of the cell medium is done by bulk diffusion through a porous material which limits the efficiency and accuracy of the dissolved gases control. This method also requires the use of materials such as silicon elastomer Polydimethylsiloxane, PDMS, that can potentially absorb small molecules limiting the correlation of dosage compounds with the effect observed in the cellular culture. The system is not suitable for culturing large biological 3D structures such as tissue biopsies due to its size, and the culturing time is limited to 3 days, maximum 5 days.

Moreover, this system is not entirely compatible with the use of standard multi-well culture plates. Finally, this system lacks the flexibility to interconnect different cell cultures and provide perfused media in the plate or to choose different configurations of wells carrying seeded cells.

Patent application WO2007092571 (WAFERGEN INC) describes a biological tissue culturing device. This device comprises: a multi-well plate comprising a plurality of wells, in which each well is shaped to receive at least two insertable tubes, a first nutrient feed manifold comprising a plurality of individual well supply lines, a manifold positioned above the wells and aligned such that each well supply line extends to a position adjacent to the bottom of a well, a second waste disposal manifold comprising a plurality of individual waste disposal lines. This second manifold extends to a position adjacent to the bottom of the wells, where the waste supply line extends to a point slightly elevated above the well supply line. In some variations, the wells may include ports connecting adjacent wells to each other, allowing the passage of materials between individual wells of the plate. A temperature controlled lid including inlets for gas diffusion is also disclosed. The ports (external input/output connection) can also include one or more valves controlling the opening/closing of the port. Sensors for dissolved Oxygen and pH incorporated in some of the wells are also disclosed.

This system has some drawbacks, in particular, the perfusion medium is fed from the outside (external actuators to move the liquid directly). The gas mixture is supplied from the outside for the enrichment of the media only by surface diffusion inside the well. Gas enrichment by delivering gas to wells through specific ports is not suitable for gas-enriched microfluidic devices. The waste is sucked from the outside, independent of the fluid supply actuators. Gases, infused liquids and waste are manipulated using independent external actuators which increases the complexity of the system. The mixing is done in a well by shaking the well outwards or by introducing stirrers of the magnetic or mechanical type, which potentially modifies the position conditions of the cells. Handling of liquids, reagents or media, infused inside is not fully contained or isolated from the external environment and leads to potential contaminations that can compromise the cell culture. The sensors to control dissolved gases and pH inside the wells are in direct contact with the cells in the wells and this limits the accurate control of the media conditions and of the effect of the cells metabolism to the media.

Patent document EP 0 263 364 A2 (SUZUKI SHOKAN KK) relates to a method for supplying culture medium to a cell culture vessel and the systems implementing this method. This method includes in particular the injection of fresh gas into the cell culture chambers of the container. This injection of gas is done by the volume of gas included in these chambers.

Patent document EP 2 623 587 (CELLIX LTD (IE)) relates to a system and a method for moving cell culture medium by pressure difference. According to this method, once the pump has drawn in a predetermined quantity of fresh medium, the pressure is equalized between the two containers. The liquid is moved by suction from the pump and by decreasing the pressure in one of the containers.

Patent document WO 2018/136752 (ESSEN BIOSCIENCE INC) relates to a device making it possible in particular to control the circulation of fluids and the atmospheric environment of a cell culture in microplates. The circulation of the fluid in a microfluidic circuit, as well as the control of the gaseous composition of the fluid is done by controlling the pressure at the level of the pneumatic cap, via one or more pneumatic regulation valves. The injection of gas to create the differential pressure is carried out by the top of the well of a microplate, that is to say by the volume of gas.

Patent document WO 2018/013646 (EMULATE INC) relates to methods for eliminating bubbles in a microfluidic device without the need to stop the flow. In particular, WO 2018/013646 discloses methods that combine pressure and flow control.

Patent document WO 2004/027016 (COOK UROLOGICAL, INC) relates to a perfusion incubator comprising a set of incubation wells. A peristaltic pump passes the culture medium through a conditioning unit before supplying all the incubation wells with this conditioned culture medium.

These currently available methods either do not provide adequate isolation from the outside, or have limitations in terms of fluidic versatility and communication between wells, or require external provisions for handling reagents during experimentation, either are unable to control gaseous enrichment of the medium, or generate shear forces and stresses on cultured cells and cell assemblies that greatly exceed physiological levels.

Gas enrichment is generally difficult to achieve in microfluidic devices and other cell culture devices where contact of the perfused liquid surface with the external gas is limited or non-existent and where diffusion mechanisms are not an effective solution.

Two particular patent applications, Patent Application WO2016076795 and US10711234, provide means of culturing cells in microfluidic devices with an optically clear gas-permeable membrane placed in their bottom allowing gas exchanges between the cell culture in a microfluidic enclosure and the gas environment present outside the device. These devices allow control of the dissolved gas and pH in the cell culturing spaces but it is entirely dependent on diffusion and the gas composition of the external gaseous environment passively in contact with the gas-permeable membrane. Also, passive diffusion will depend on the velocity of the flown liquid making it difficult to make a standard procedure for any kind of wished flow in typical microfluidic setups ranging from 1 nl per minute to 10 ml per minute.

A possible solution to this problem is the patent document EP3712244 (Cherry Biotech SAS) which concerns a system and a process for moving a fluid and simultaneously gas-enriching a liquid cell culture medium. Depending on this process, a controlled volume of a gas or gas mixture is injected inside at least one reservoir or one well containing a volume of liquid by means of at least one gas flow controller. Said injection produces: bubbling and agitation of said volume of liquid, an accumulation of said gas or mixture of gas by buoyancy in an airtight space formed by said volume of liquid and said at least one reservoir or well, and an increase in pressure in said at least one reservoir or well until reaching sufficient controlled pressure less than or equal to 10 bar, said increase producing a displacement of said liquid volume per at least one outlet fluid connecting said volume of liquid and the exterior of the said at least one reservoir or well.

This automated microfluidic cell culture system for controlling the *in vitro* culture environment allows fine control of cell culture conditions, to approximate *in vivo* conditions as closely as possible. This system is a combination of controlled perfused media of adequate chemical, biochemical and gaseous composition, and a controlled temperature. This microfluidic cell culture system makes it possible to guarantee the control of the shear stress of the cultured cells, as well as the avoidance of leaks of biological elements and/or sensitive and/or dangerous compounds and/or contamination by foreign germs or unwanted particles from samples when performing experimental protocols.

However, this method presents a strong dependence of the times for achieving controlled changes of media dissolved gas(es) and/or pH on the volumes of liquid contained on the media reservoirs and on the flow applied to the gas and ultimately the liquid. It also has limited control of the achieved values for dissolved gas(es) and/or pH due to the intrinsic dependence of the method to the original media compositions.

There is therefore a need for a much more versatile technique and apparatus adaptable to, but not limited to, the standard of multi-well culture plates for biological entities, such as tissue, spheroids, organoids or cells, confinement in highly controlled cell culture environments, in terms of temperature, medium composition, gas(es) composition, fluid displacement and shear stress. Additionally, there is a need for isolation from outside of these controlled environments within multi-well culture plates while allowing optical observation and selective communication between different confined wells of the multi-wells plate when necessary.

### Disclosure of the Invention

The invention fulfils this need by proposing a method for controlling fluid displacement and dissolved gas(es) composition and/or pH of a volume of liquid cell culture medium contained in at least one closed chamber. The method comprises:
- an injection of a gas, or gas mixture by at least one gas controller,
- an injection of a liquid cell culture medium by at least one liquid controller into said at least one closed chamber,
- a production of a diffusion and pressure driven exchange of gas, or gas mixtures, between said injected liquid cell culture medium and said injected gas, or gas mixture through at least one gas-permeable membrane,
- a collection of measurements of a dissolved gas(es) concentration and/or pH of said liquid cell culture medium by at least one dissolved gas(es) concentration and/or pH sensor,
- a determination of an injection flow rate and/or pressure of said gas, or gas mixture, and/or gas mixture composition, and/or an injection flow rate and/or pressure of said liquid cell culture medium based on said measured dissolved gas(es) concentration and/or pH of said liquid cell culture medium, fluid flow rate to be less or equal to 50ml/min, local pressures to be less or equal to 100 bar and a predetermined dissolved gas, or gas mixture, composition and/or pH of the liquid cell culture medium,
- an adjustment of an injection flow rate and/or pressure of said gas, or gas mixture by said at least one gas controller and/or an injection flow rate and/or pressure of said liquid cell culture medium by said at least one liquid controller based on said determination to obtain said predetermined dissolved gas, or gas mixture composition and/or pH of the liquid cell culture medium.

Advantageously, the method according to the invention makes it possible to maintain an ideal environment for cell culture in a closed environment that totally isolates any biological entities such as: cell cultures, spheroids, tissues, organoids, cells in extracellular matrixes, cells in hydrogels, etc. from the external environment.

More particularly, the method allows to precisely control the gas(es) composition and/or pH of a liquid, such as a cell culture medium. In other words, the dissolved gas(es) composition and/or pH of the liquid cell culture medium is always adapted to the experimental needs for the cultured biological sample(s).

By "gas(es) composition" we mean in the following description, the nature and quantity of the gas(es). For example, when the gas flow injected is a gas mixture, the composition comprises the different types of gas in the mixture and their respective quantities (i.e., concentration of each gas and/or percentage of each gas contain in the total gas flow) within the total injected gas flow.

Such a gas(es) containing liquid is then transferred into a closed chamber which is a hermetically sealed environment in which biological entities can be cultured and studied.

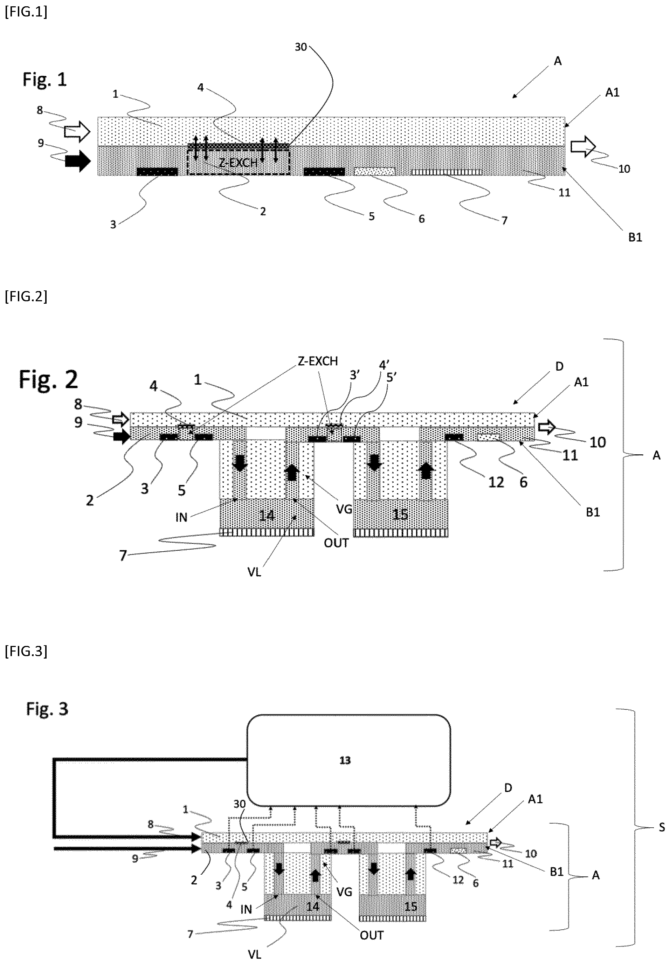

By "closed chamber" we mean in the following description several possible material arrangements such as a closed microfluidic channel of a microfluidic device (see for example Figure 1) or a reservoir integrated in a microfluidic device or sealed by a microfluidic device (see for example Figures 2 to 5), thus forming a closed chamber of a fluidic device or fluidic assembly. The reservoir can be integrated in a microfluidic channel (for example by forming a space to act as a reservoir using 3D printing), an isolated reservoir (for example petri dish or a single well of a multi-well cell culture plate) or several reservoirs mounted in parallel and/or in series (for example several well of a multi-well cell culture plate).

Thus, it is possible to cultivate biological samples within this closed chamber thanks to a continuous renewal of the culture medium comprising gas(es) without contact with no other external environment than the exchanging gas(es) and liquid.

Furthermore, the method according to the invention allows to precisely control the liquid flow in order to allow the selective application of shear stresses on the biological sample(s). Shear stress is the tangential force of the flowing cell culture media on the cells surface and has important physiological and physio pathological implications.

For example, by fixing the liquid flow in a microfluidic cell culture system and applying this method by just adjusting the injected gas flow rate and/or pressure, the cell culturing media velocity at the biological entities or cell culture to liquid interface is controlled by the closed chamber geometry, thus allowing the selective application of shear stresses.

For this, the gas or gas mixture, and the liquid (such as a cell culture medium) is injected by dedicated manual or computer-controlled controllers into a microfluidic cell culture system.

In order to adjust in real time the dissolved gas(es) composition and/or the pH in the liquid, the liquid cell culture medium is in contact with the gas, or gas mixture, only through at least one gas-permeable membrane. Through diffusion principle, some of the flowing gas(es) and dissolved gases are pass through the gas-permeable membrane to modify the gas(es) composition of the liquid cell culture medium. This liquid then flows to the closed chamber containing biological entity(ies).

The dissolved gas(es) concentration and/or pH is then measured by sensor(s) and the data transmitted to a control module that display it on a human-machine interface or alternatively process it. Thus, it is possible to know the gas(es) composition (i.e., nature of the gas(es) and concentration or percentage of each gas in a total gas flow) of the liquid (i.e liquid cell culture medium) in real time and thus make adjustment(s) if necessary. For example, for controlling a gas or gas mixture diffusing in the liquid cell culture medium, the dissolved gas(es) concentration and/or pH sensor(s) allows to known in real time the nature of gas(es) in the gas flow and the percentage of each gas and thus to allow the adjustment of the gas(es) composition via the adjustment of the gas, or gas mixture, injection flow rate and/or pressure.

Hence it is not necessary to use an incubator structure to guarantee the biological living conditions in a microfluidic cell culture system. Furthermore, accurate values for different dissolved gas(es) concentrations can be achieved emulating the nature-found values occurring for the biological entity cultures at different physiological and physio pathological conditions. For example, it is well known the intra and inter organ variability of pH and dissolved oxygen in the human body.

The method according to the invention enables a cell culture, a tissue or any other living matter to be maintained in a living condition by a precise control of the vital biological parameters such as the composition in gas(es) of the liquid cell culture medium and pH, and allows precise control of cell shear stress through low perfusion flows to prevent viability from being compromised or to achieve specific cell or biological entities phenotypes.

According to a particular aspect of the invention, said collection of measurements of the dissolved gas(es) concentration and/or pH of said liquid cell culture medium is done after the production of the diffusion and pressure driven exchange of gas, or gas mixtures, between said liquid cell culture medium and the gas, or gas mixture through said at least one gas-permeable membrane.

Advantageously, it is possible to know the gas(es) composition and/or pH of a liquid, such as a cell culture medium, directly after the gas(es) composition and/or pH of the liquid cell culture medium has been modified thanks to the gas-permeable membrane, or series of gas-permeable membranes, and the diffusion principal.

Thus, it is possible to verify the composition of gas(es) and/or the pH of the liquid cell culture medium before it enters a closed chamber comprising biological sample in culture and adjust it if necessary.

According to a particular aspect of the invention, said method further comprises a collection of measurements of dissolved gas(es) concentration and/or pH of said liquid cell culture medium before the production of the diffusion and pressure driven exchange of gas, or gas mixtures, between said liquid cell culture medium and the gas, or gas mixture through said at least one gas-permeable membrane.

Advantageously, it is possible to know the dissolved gas(es) composition and/or the pH of a liquid, such as a cell culture medium before the gas(es) composition and/or pH has been modified thanks to the gas-permeable membrane and the diffusion principal.

Thus, it is possible to make a comparison of the dissolved gas(es) composition and/or pH of the liquid cell culture medium before and after it comes into contact with the gas-permeable membrane, or series of gas-permeable membranes, and then verify the gas(es) composition and/or pH, and even adjust it if necessary before it comes into contact with any biological entity in the closed chamber.

According to a particular aspect of the invention, said method further comprises a collection of measurements of the dissolved gas(es) concentration and/or pH of said liquid cell culture medium after said liquid cell culture medium has passed through at least one biological entity cultured in the at least one closed chamber.

Advantageously, it is possible to know the gas(es) composition and/or pH of the liquid cell culture medium after it passes through a closed chamber containing a biological entity(ies) in cultivation or after a series of closed chambers containing the same or different biological entities.

It is thus possible to detect changes in composition related to the biological entity, for example, what type of gas(es) has been consumed by it and in what quantity (for example oxygen). It is even possible to detect the production of a type of gas(es) by the biological entity (e.g., carbon dioxide). In another example, it is possible to know the pH modification link to the production or consumption of gas(es) and/or metabolites.

According to a particular aspect of the invention, said determination uses a feed-back loop automated system based on a mathematical algorithm and/or an artificial intelligence module implemented by a control module.

Advantageously, the method according to the invention allows to adjust in real time and in an automated manner the gas(es) composition and/or pH of a liquid cell culture medium.

More particularly, as described above, with the help of dissolved gas(es) concentration and/or pH sensors positioned only after, or before and after the gas-permeable membrane, it is possible to know in real time the gas(es) composition and/or pH of the liquid cell culture medium. The measurements are transmitted from the sensor(s) to the control module. The control module can then process the received data from the sensor(s) and compare it to a predetermine dissolves gas(es) composition and/ pH value to be obtain (for example specific to the biological entity in cultivation or to the biological experiment implemented). For this, the control module uses a feedback loop based for example on a mathematical algorithm and/or an artificial intelligence module. In order to have a precise shear stress adapted to the biological entity cultured, the control module also takes into account that fluid flow rates should be less or equal to 50ml/min and local pressures should be less or equal to 100 bar within some of the microchannels of the microfluidic device, fluidic device or fluidic assembly.

Thus, the control module can calculate the injection flow rate and/or pressure of the gas, or gas mixture and/or the injection flow rate and/or pressure of the liquid cell culture medium to be applied to achieve the ideal gas(es) composition and/or pH of the liquid cell culture medium, fluid flow rate and local pressure.

For this, in on embodiment, the control module then transmits a command to adjust the gas(es) composition and/or pH in real time to the different flow controllers which adjust the gas(es) and/or liquid injection flow rate and/or pressure, iteratively or not, to reach the predetermine gas(es) composition and/or pH, fluid flow rate and local pressure.

In a variant, the control module then displays on a human-machine interface the result(s) to an operator that will manually adjust the gas(es) composition and/or pH in real time using the different flow controllers which adjust the gas(es) and/or liquid injection flow rate and/or pressure to reach the predetermine gas(es) composition and/or pH, fluid flow rate and local pressure.

The percentages of gases or the partial pressures and/or flows of gases composing the flown gas mix can be similarly adjusted.

According to a particular aspect of the invention, said method further comprises: measuring and/or identifying, by at least one other sensor (6) measuring before or/and after the biological entity(es), at least one physicochemical parameter of said liquid cell culture medium (2), said at least one physicochemical parameter being chosen from:
- temperature,
- metabolite(s),
- nutrient(s),
- protein(s)
- nucleic acid(s).

Thus, it is possible to maintain a cell culture, a tissue, or any other living material in a living condition thanks to precise control of the vital biological parameters such as the composition of nutrients and metabolites in the medium, the gas(es) composition, the pH and the temperature.

Furthermore, the method allows to analyze the liquid composition by identifying and/or measuring the quantity of different physicochemical parameters. For example, it is possible to know which metabolite are produced by the biological entity and in what quantity. In another example it is possible to know which nutrient are consumed by the biological entity and in what quantity. In another example it is also possible to do differential measurements between at least two identical sensors placed before and after a closed chamber containing a biological entity in cultivation to monitor physicochemical changes due to the biological entity activity for example lactate or glucose changes.

According to a particular aspect of the invention, the method further comprises a control of a temperature by injecting a thermalization liquid.

Advantageously, this fluid is fluidically isolated from the device's other microfluidic circuits.

Hence, it is possible to also integrate the temperature control of the biological cell culture environment by circulating one or more thermalization solutions in the device according to the invention (isolated from the components tested, mediums and biological entities). It is also possible to maintain different parts of the multi-well plate at different temperatures guaranteeing the integrity of the samples and of the compounds for certain experiments (drugs are sometimes kept at lower temperatures before application). The temperature control could be integrated and implemented by a control module.

According to a particular aspect of the invention, the method further comprises a successive displacement by the liquid controller of said volume of liquid cell culture medium (VL) from said at least one closed chamber to at least one other closed chamber, said closed chambers (14, 15) being mounted in series and/or in parallel.

Advantageously, the method according to the invention allows to displace a fluid with a specific gas(es) composition and/or pH (such as a liquid cell culture medium). More particularly, the flows of liquid are totally isolated from the external environment, thus guaranteeing the integrity of the biological entity cultured.

It is thus possible to regularly renew the culture medium inside a closed chamber. The composition of gas(es), nutrients, pH etc. of the culture medium is always optimal for the culture of biological entity.

By circulating the liquid cell culture medium from one closed chamber to another, it is also possible to observe the effect of the production of biological elements in the culture medium by cells, organoid, tissue etc. on other cells, organoid, tissue. Alternatively, or complementarily, it is also possible to observe the effect of circulating cells, depleted from the biological element in one closed chamber to another biological element in another closed chamber (e.g. circulating tumor cells).

The method according to the invention enables the liquid to be selectively displaced from one closed chamber to another to perfuse mediums, apply a compound to a cells' assembly or a given cell culture or to enable the communication between different assembled cells or in non-organized cell cultures, to recreate multi-organoid communications in the biological systems.

According to a particular aspect of the invention, said at least one gas-permeable membrane is made of a polymer with a thickness lower than 500µm and with bulk density lower than 1g/cm³ or any other material with specific gas permeabilities higher than 10 Barrer.

Transport properties of gases are known to depend on structural parameters like packing density in the polymer, polar group density, chain rigidity, and molecular mobility (Kulkarni, Bull. Mater. Sci., Vol. 17, No. 7, December 1994, pp. 1307-1315 ).

According to a particular aspect of the invention, said polymer is selected from the group comprising: polymethylpentene (PMP), poly(1-trimethylsilyl-1-propyne) (PTMSP), polymethylated polymers, 2,2-bistrifluoromethyl-4,5-difluoro-1,3-dioxole (PDD) and tetrafluoroethylene (TFE) copolymers.

The invention also relates to a cell culture system. The cell culture system implements the method described previously. The cell culture system comprises:
- A microfluidic device (A, D), a fluidic device (A) or fluidic assembly (A), called device (A, D), said device (A, D) comprising:
   - at least two microfluidic circuits each comprising at least one microfluidic channel, one of said at least two microfluidic circuits being able to contain a flowing gas, or gas mixture, or sequence of gases injected by a gas controller, and at least one other of said at least two microfluidic circuits being able to contain a flowing liquid, or sequence or different liquid cell culture medium injected by a liquid controller,
   - at least one gas-permeable membrane being able to produce a diffusion and pressure driven exchange of gas, or gas mixtures, between a liquid cell culture medium circulating in one of the at least two microfluidic circuit and a gas, or gas mixture circulating in the other of the at least two microfluidic circuit, said at least two microfluidic circuits overlapping with each other *via* the at least one gas-permeable membrane connecting at least one microfluidic channel of each at least two microfluidic circuits to each other,
   - at least one dissolved gas(es) concentration and/or pH sensor placed in the microfluidic circuit in which the liquid cell culture medium is circulating and configured to collect measures of a dissolved gas(es) concentration and/or pH of said liquid cell culture medium;
   - at least one closed chamber;
- at least one gas controller configured to inject said gas, or gas mixture into said device (A, D);
- at least one liquid controller configured to inject said liquid cell culture medium into said closed chamber of said device (A, D);
- said at least one gas controller and at least one liquid controller being configured to adjust an injection flow rate and/or pressure of said gas, or gas mixture, or a gas mixture composition, and/or an injection flow rate and/or pressure of said liquid cell culture medium, based on said measured dissolved gas(es) concentration and/or pH of said liquid cell culture medium, fluid flow rates to be less or equal to 50ml/min, local pressures to be less or equal to 100 bar and a predetermined dissolved gas, or gas mixture, composition and/or pH of the liquid cell culture medium.

Advantageously, the cell culture system according to the invention makes it possible to create a closed microfluidic cell culture system that totally isolated any biological samples: spheroids, tissues, organoids, extracellular matrix cells, hydrogel cells, etc. from the external environment. More particularly, the cell culture system allows to precisely control the gas(es) composition and/or pH of a liquid, such as a cell culture medium, circulating in a microfluidic circuit of a microfluidic device, fluidic device or fluidic assembly. The microfluidic device, fluidic device and fluidic assembly are called hereinafter "device".

Such a liquid is then transferred into a closed chamber formed by a reservoir (i.e, isolated reservoir or a well of a multi-well culture plate) and by the device. It is thus possible to cultivate biological samples within this closed chamber thanks to a continuous renewal of the culture medium with a precise gas(es) composition and/or pH without contact with the external environment.

For this, the device according to the invention comprises several different microfluidic circuits that comprise one or more microfluidic channels. It is thus possible to circulate through these channels either one or more gases, or gases mixtures, or liquids such as a cell culture medium. For this purpose, controllers connected to the device inject into these microfluidic circuits either a gas, or gas mixture, or a liquid that will circulate continuously. These controllers are typically gas controller or liquid controller configured to inject a gas (or gas mixture), control a gas mixture composition, and/or inject a liquid continuously in the appropriate microfluidic circuit (i.e., either a microfluidic circuit dedicated to the circulation of gas(es) or gases mixtures or to the circulation of liquid, such as culture cell medium) while controlling in real time pressure and/or injection rate of the gas(es) or liquid.

When the fluid is a liquid, such as cell culture medium, then it passes through one or several closed chambers containing, for example, biological samples in culture. The liquid then is evacuated from the closed chamber to go to another closed chamber or to the outside of the device.

On the contrary, when the fluid is a gas or gas mixture, it circulates in a microfluidic circuit to the outside of the device.

In order to modify the gas(es) composition and/or the pH of the liquid, the different microfluidic channels of the microfluidic circuits are in contact only through at least one gas-permeable membrane. Indeed, this gas-permeable membrane connects on the one hand a microfluidic circuit of the device containing flowing gas(es) and on the other hand a microfluidic circuit of the device containing a flowing liquid, such as a cell culture medium. Through diffusion and/or local pressure, a volume of the flowing gas(es) within the dedicated microfluidic circuit pass through the gas-permeable membrane to modify the liquid cell culture medium flowing in the microfluidic circuit dedicated to the liquid cell culture medium. Alternatively, or in addition, gas(es) contains in the liquid cell culture medium can also passes through the gas-permeable membrane to the microfluidic circuit containing the flowing gas(es).

In other words, liquid and gas(es) that circulate within the microfluidic circuits can only exchange gas molecules at this gas-permeable membrane. Thus, the device allows to modify the gas(es) composition and/or pH of a liquid, such as a cell culture medium, using the diffusion and/or local pressure principal through the gas-permeable membrane. The liquid with a precise gas(es) composition and/or pH then flows to the closed chamber containing biological sample(s).

According to another aspect of the invention, when said cell culture system comprises a fluidic assembly (A), then said cell culture system further comprises:
- a multi-well cell culture plate comprising a plurality of wells,
- said fluidic assembly (A) comprising a microfluidic device (D) mounted on said multi-well cell culture plate,
and said microfluidic device (D) further comprises:
- a fluids-routing layer comprising at least one connection orifice or at least one fluidic connection duct allowing the attachment of at least one fluid connector coming from said at least one gas controller and/or said at least one liquid controller,
- a manifold or liquid-routing layer forming the base of the microfluidic device (D), said manifold comprising at least two nozzles or orifices or ducts, and at least one cavity, said at least one cavity forming with at least one well of said multi-well cell culture plate said at least one closed chamber containing a volume of liquid cell culture medium, and said at least two nozzles or orifices or ducts forming at least one liquid inlet and at least one liquid outlet connecting the volume of liquid cell culture medium contained in the at least one closed chamber to an exterior of said at least one closed chamber,
- said at least two microfluidic circuits each comprising at least one microfluidic channel, said at least two microfluidic circuit extending from at least one connection orifice or at least one fluidic connection duct of said fluids-routing layer to a fluid outlet of said microfluidic device (D) or to said at least two nozzles or orifices or ducts of said manifold, *via* said at least one microfluidic channel, one of said at least two microfluidic circuits being able to contain the flowing gas, or gas mixture continuously injected by said gas controller, and at least one other of said at least two microfluidic circuits being able to contain said flowing liquid cell culture medium continuously injected by said liquid controller,
- said at least two microfluidic circuits overlapping with each other *via* the at least one gas-permeable membrane connecting at least one microfluidic channel of each at least two microfluidic circuits to each other,
- the at least one dissolved gas(es) concentration and/or pH sensors are embedded inside said at least one microfluidic channel of the at least one of the at least two microfluidic circuits in which the liquid cell culture medium is flowing after or before and after the gas-permeable membrane.

The cell culture system according to the invention is advantageously compatible with any type of disposable multi-well plates. These multi-well plates are a standard widely used in pharmaceutical, biotechnological and life sciences experiments.

Hence the cell culture system according to the invention is simple to use whatever the embodiment and can be easily adapted to any standard multi-well plates. The cells cultured in the multi-well plate are perfectly isolated from the exterior environment and the changes of nutrient medium and the treatment changes no longer require to be manipulated manually. The gas(es) composition and/or pH modifications of the cell culture medium is automatically controlled at any cell culture media flow rate, which not only eliminates the need for an incubator, and incubator environment exchanges, but also confers superior gas exchanging capabilities.

Furthermore, the assembly allows safe recovery of the sample after experimentation from the multiwell plate after separating the microfluidic device D from the multiwell plate.

According to another aspect of the invention, said at least one dissolved gas(es) concentration and/or pH sensor(s) collects measures of the dissolved gas(es) concentration and/or pH of said liquid cell culture medium after the production of the diffusion and pressure driven exchange of gas, or gas mixtures, between said liquid cell culture medium and the gas, or gas mixture through said at least one gas-permeable membrane.

According to another aspect of the invention, said device (A, D) further comprises at least one dissolved gas(es) concentration and/or pH sensor configured to collect measurements of dissolved gas(es) concentration and/or pH of said liquid cell culture medium before the production of the diffusion and pressure driven exchange of gas, or gas mixtures, between said liquid cell culture medium and the gas, or gas mixture through said at least one gas-permeable membrane.

According to another aspect of the invention, said device (A, D) further comprises at least one dissolved gas(es) concentration and/or pH sensor configured to collect measurements of dissolved gas(es) concentration and/or pH of said liquid cell culture medium after said liquid cell culture medium has passed through at least one biological entity culture in the at least one closed chamber.

According to another aspect of the invention, the cell culture system further comprises a control module configured to display on a human-machine interface the data collected by the at least one dissolved gas(es) concentration and/or pH sensor(s).

According to another aspect of the invention, the cell culture system further comprises a control module configured to:
- determine the injection flow rate and/or pressure of said gas, or gas mixture and/or the injection flow rate and/or pressure of said liquid cell culture medium based on said dissolved gas(es) concentration and/or pH of said liquid cell culture medium measurements, fluid flow rates to be less or equal to 50ml/min, local pressures to be less or equal to 100 bar and predetermined dissolved gas, or gas mixture, composition and/or pH of the liquid cell culture medium, using a feed-back loop automated system based on a mathematical algorithm and/or an artificial intelligence module implement by the control module;
- transmit a command to adjust the injection flow rate and/or pressure of said gas, or gas mixture to the gas controller and/or the injection flow rate and/or pressure of said liquid cell culture medium to the liquid controller.

According to another aspect of the invention, said device (A, D) comprises at least one other sensor being able to measure and/or identify at least one physicochemical parameter of said liquid cell culture medium, said at least one physicochemical parameter being chosen from:
- temperature,
- metabolite(s),
- nutrient(s),
- protein(s),
- nucleotide acid(s).

According to another aspect of the invention, said device (A, D) comprises at least one other parallel microfluidic circuit, having at least one microfluidic channel in which an injected thermalization liquid flows into.

According to another aspect of the invention, said at least one microfluidic circuit containing the flowing liquid cell culture medium fluidically connects the volume of liquid cell culture medium) contained in said at least one closed chamber to another volume of liquid cell culture medium contained in at least one other closed chamber, said volume of liquid cell culture medium being successively displaced by said at least one liquid controller from said at least one closed chamber to said at least one other closed chamber through said at least one microfluidic circuits.

### List of figures

Other purposes, characteristics and advantages of the invention will emerge more clearly upon reading the following description of a particular embodiment, provided as a simple non-restrictive example, in relation to the figures, among which:
**[****fig. 1]:** Figure 1 presents a diagram of an example of an embodiment of a microfluidic device A suitable for implementing the method according to the invention.
**[****fig. 2]:** Figure 2 presents a diagram of an example of an embodiment of a fluidic device or a fluidic assembly A suitable for implementing the method according to the invention.
**[****fig. 3]:** Figure 3 presents a diagram of an example of a cell culture system comprising the fluidic device or the fluidic assembly A according to Figure 2 and implementing the method according to the invention.
**[****fig. 4A]:** Figure 4A illustrates an exploded view diagram of the structure of an example of the embodiment of the fluidic assembly A constructed by assembly of a multi well cell culture plate and a complementing microfluidic device D according to invention and thus suitable for implementing the method according to the invention;
**[****fig. 4B]:** Figure 4B illustrates an example of overlap of the microfluidic circuits for gas and for liquid of the fluidic device or fluid assembly according to Figures 2 to 4A and implementing the method according to the invention;
**[****fig. 5]:** Figure 5 illustrates a view of a fluidic assembly A of a multi-well cell culture plate with a microfluidic device D according to Figures 4A and 4B.

### Detailed description of the embodiments of the invention

The general principle of the invention consists in confining and controlling in a temporal and spatial manner the chemical, biochemical and physical properties, such as the dissolved gas(es) composition, pH, the induced shear stresses or the local temperatures, of biological environments within devices, such as for example bioreactors, fluidic or microfluidic devices, or multi-well cell culture plates, also called multi-well culture plates. More particularly, the invention seeks to allow an automatic control of the cell culture conditions using a specially designed device integrated into a cell culture system.

In particular, the invention provides an effective way to accurately control, in a cell culture system comprising fluidic or microfluidic device:
- the gas(es) composition, i.e the nature of the gas(es) and amount of dissolved gas(es), contained in moving fluids, such as a liquid cell culture medium;
- pH of a liquid cell culture medium;
- the local shear stress applied to cultured biological entities.

For this, the invention allows to control the gas (or gas mixture) flow rate and/or the pressure (or partial pressure in the case of gas mixture), and thus the desired dissolved gas(es) composition, pH and shear stress on biological entities.

The invention is useful for conducting controlled biological assays in the fields of pharmacological and biotechnology discovery, controlled cell differentiation, therapeutic and cosmetic assays, compound research and assays, and custom assays, as well as in all life science experiments requiring precise control of the environmental conditions of cells, tissues or organoids.

More particularly, the invention proposes to displace a liquid, such as cell culture medium, and simultaneously to modify in real time this liquid gas(es) composition and/or pH, before feeding it to a biological entity. This preliminary stage of dissolved gas(es) composition modification of a culture medium makes it possible to maintain the cell culture conditions over the long-term (i.e., between 1 day and 60 days) and to apply specific physiological or physio pathological conditions (for example pH, dissolved Oxygen concentration...etc.) to this cell culture in a time-dependent manner. In other words, the microfluidic device, fluidic device or fluidic assembly and cell culture system according to the invention allows to maintain a stable and time-dependent cell culture environment including: gas(es) composition of the liquid cell culture medium, temperature, shear stress, pH (due to its well-known dependence on dissolved CO₂ in aqueous solutions) ...etc.

Subsequently, for the purposes of simplification, "biological entity(ies)" is understood to mean a non-organised or organised cell culture, namely a cell assembly such as for example tissues, spheroids or organoids. These cells can for example be from animals, such as humans, mice, or plants.

"Multi-well culture plate" is understood to mean standard plates comprising at least six wells in which a specific nutrient culture medium, certain chemical or biochemical compounds, with the cell type studied is introduced, as well as cells to be cultured.

"Microfluidic channel" or "channel" is understood to mean channels with cross sections between 0.1µm² and 25mm².

**Figure 1** shows a diagram of an example of an embodiment of a microfluidic device suitable for implementing the method according to the invention. The method according to the invention will be described in relation to **Figure 3** below.

The microfluidic device A comprises at least two microfluidic circuits.

By microfluidic circuit (also called circuit in the following description) it is meant a circuit comprising:
- at least one inlet for a fluid, such as a gas, or gas mixture, or a liquid (not shown),
- at least one microfluidic channel (also called channel in the following description) running across the microfluidic device A,
- at least one fluid outlet 10.

The inlet for fluid is, for example, a fluid connection orifice or fluidic connection duct (not shown) allowing the attachment of a fluid connector (not shown) coming from a flow controller that supplies a fluid. The flow controller controls the fluid pressure and/or its flow rate in real time. This control of pressure and/or flow rate values by the various flow controllers can be done manually, i.e. an operator will modify the pressure and/or flow rate values. Alternatively, this control of pressure and/or flow rate values by the various flow controllers can be carried out automatically, i.e. without the operator's intervention.

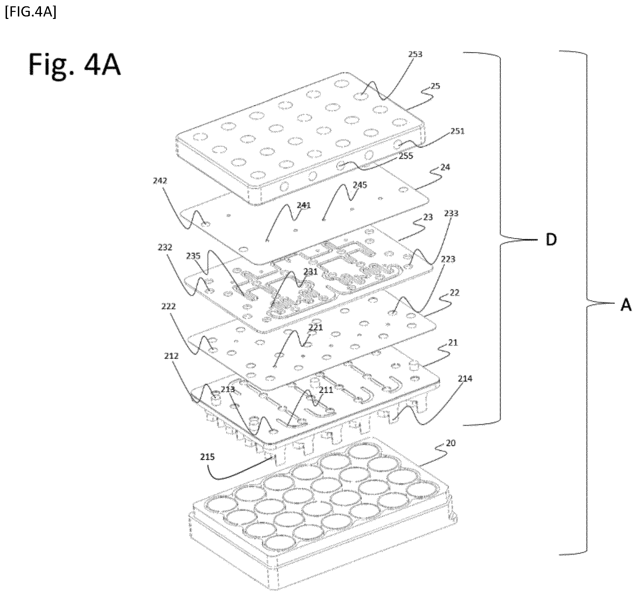

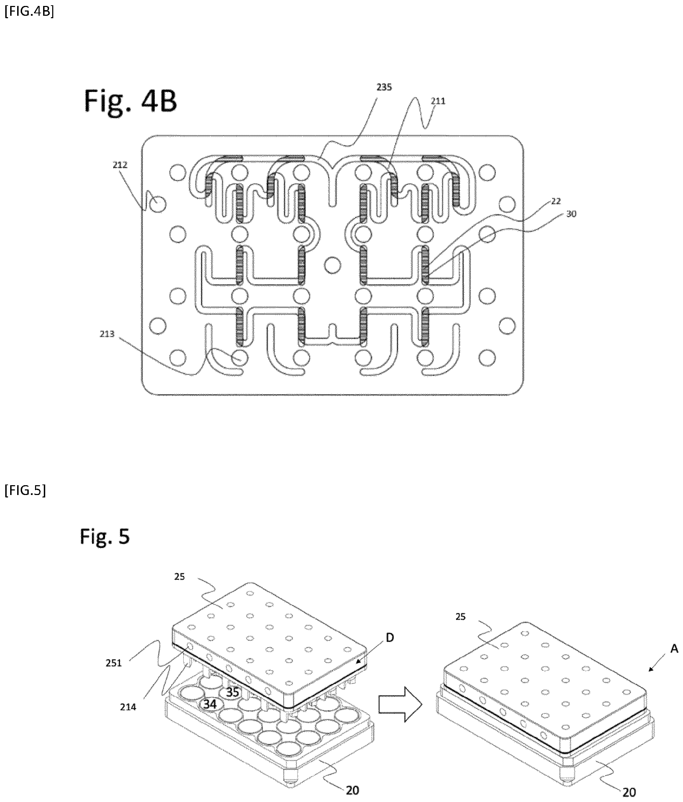

The microfluidic channel(s) extends through the microfluidic device A in a continuous manner (see **Figure 1** for example), and/or in a discontinuous manner, thus forming in this case a fluidic device or fluidic assembly A, as shown in relation with **Figures 2****,** **4A****,** **4B****.** Furthermore, the microfluidics channels run through the microfluidic device A by being superimposed on each other in a parallel manner, i.e., they follow the same path within the microfluidic device A. Alternatively, or in addition, each microfluidic channel follows their own path and will only cross each other in one or more particular intersections, called overlapping liquid and gas microfluidic channels zone 30 (or overlapping zone 30), as shown in **Figure 4B****.**

The fluid outlet 10 allows the fluid (gas or liquid) to be evacuated from the microfluidic device A to a waste or sample recovery container (not shown), for example. In an embodiment, the fluid outlet 10 is common to the microfluidic circuit(s) in which the liquid flows and the microfluidic circuit(s) in which the gas(es) flows. In a variant, each microfluidic circuit has its one fluid outlet 10.

In order to simplify the description of this embodiment of the microfluidic device A according to the invention, **Figure 1** represents a microfluidic device A comprising two microfluidic circuits. However, it should be noted that the microfluidic device A can have more than two microfluidic circuits. Moreover, in **Figure 1****,** each microfluidic circuit comprises a single microfluidic channel, but it should be noted that each circuit can comprise several microfluidic channels in different configuration (for example one channel can separate to form two different channels, and conversely two channels can be joined together to form one).

The microfluidic device A according to **Figure 1** comprises a first microfluidic circuit comprising a microfluidic channel A1 and a second microfluidic circuit comprising a microfluidic channel B1. In each of the microfluidic channels A1 and B1, a fluid, such as a gas (or gas mixture) or a liquid, can circulate. In other words, each microfluidic circuit is dedicated to the circulation of either a gas (or mixture of gases) or a liquid. The liquid can be any liquid needed for biological experiments or it can be a mix of different liquid. Throughout the description the liquid is a cell culture medium suitable for culturing biological entities.

In **Figure 1****,** a fluid such as a gas, or gas mixture 1, flows as a stream or total gas flow 8 through the microfluidic channel A1. The gas can be any gas suitable for biological entity culture, such as O₂, CO₂ and N₂. The gas mixture comprises different percentages of O₂, CO₂ and N₂, for example.

In particular, the gas, or gas mixture 1, is injected by a manually or computer-control controller, called gas flow controller, *via* one or more fluid connection orifice(s) or fluidic connection duct(s) (not shown) allowing the attachment of at least one fluid connector (not shown) coming from the gas flow controller. This gas flow controller externally supplies the gas or gas mixture 1 to the flow 8. The gas flow controller is configured to control: the gas(es) composition (i.e nature of the gas(es) and quantity), the gas(es) pressure (or partial pressure when the gas is a gas mixture) and/or the gas(es) flow rate in real time. When the gas is a gas mixture, the gas flow controller can be configured to control the quantity of each gas in the total flow 8, i.e the percentage of each gas of the mixture (for example O₂, CO₂ and N₂) in the total gas flow 8 and the partial pressure of each gas of the mixture in the total gas flow 8.

In the microfluidic channel B1, a fluid such as a liquid 2 flows as a stream or flow 9. In particular, this liquid 2, is injected by a manually or computer-control controller, called liquid flow controller, *via* one or more fluid connection orifice(s) or fluidic connection duct(s) (not shown) allowing the attachment of at least one fluid connector (not shown) from the liquid flow controller. The liquid flow controller externally supplies a liquid 2, such as a liquid cell culture medium to the flow 9. The liquid flow controller controls the pressure of the liquid 2 and/or its flow rate in real time. Indirectly, in a microfluidic device A containing a biological entity 7 it can be used to control the shear-stress and/or pressure applied to the biological entity 7 and its cells.

The two microfluidic channels A1 and B1 include a gas-permeable membrane 4 at an intersection or overlapping liquid and gas microfluidic channels zone 30 (also called overlapping zone 30) (see **Figure 4B**). In other words, the channels A1 and B1 are independent on part of their path, but communicate together at a gas-permeable membrane 4. It is thus possible to change the composition of dissolved gas(es) in the liquid 2 circulating in the channel B1 thanks to the principle of gas diffusion and local pressure through this gas-permeable membrane 4. For example, at the level of the overlapping zone 30 the liquid 2 can be gas(es) enriched or gas(es) depleted depending on the partial pressure's equilibrium. Indeed, for example, the inventors have observed dissolved oxygen removal when the liquid is placed in contact with an oxygen free environment (e.g. only nitrogen).

It should be noted that the different fluid flows can run parallel to each other in the same or opposite directions. They can also flow crosswise to one another.

This gas-permeable membrane 4 can be made of a polymer with a thickness lower than 500µm and with bulk density lower than 1g/cm³. In particular, the polymer can be selected from the group comprising: polymethylpentene (PMP), poly(1-trimethylsilyl-1-propyne) (PTMSP), polymethylated polymers, 2,2-bistrifluoromethyl-4,5-difluoro-1,3-dioxole (PDD) and tetrafluoroethylene (TFE) copolymers.

Alternatively, this gas-permeable membrane 4 is made of any other material of similar properties with permeabilities higher than 10 Barrer for the gases of interest for the gas-liquid exchange (e.g. O₂, CO₂). Indeed, it is well-known that gas transport through gas-permeable membrane towards liquid will depend on Henry's and Fick's laws, which are governed by the gases partial pressures, diffusion coefficients and membrane permeability coefficients.

The channel B1, in which the liquid 2 circulates, also comprises a dissolved gas(es) concentration and/or pH sensor 5 configured to collect measure(s) of the dissolved gas(es) concentration and/or the pH of the liquid 2. These sensors are also configured to transmit the collected data to a control module 13 for example, or another module suitable to display on an interface human-machine the data (not shown in **Figure 1****,** see for example **Figure 3**). In a variant, the dissolved gas(es) concentration and/or pH sensor 5 is also able to identify the nature of the dissolved gas(es).

Advantageously, this dissolved gas(es) concentration and/or pH sensor 5 is placed after the gas-permeable membrane 4. In this way, it is possible to verify the dissolved gas(es) composition and/or pH of the liquid 2 after the exchange of gas(es) molecules between the gas flow 8 and the liquid flow 9. In a variant, when a gas or mixture of gases is injected in the channel A1, the dissolved gas(es) concentration and/or pH sensor 5 comprises a plurality of dissolved gas(es) concentration and/or pH sensors configured to measure the concentration, or the percentage, of each gas of the mixture in the liquid 2 and/or its pH.

In a variant, the channel B1, in which the liquid 2 circulates, comprises another dissolved gas(es) concentration and/or pH sensor 3 placed before the gas-permeable membrane 4 and configured to collect measure(s) of the dissolved gas(es) concentration and/or the pH of the liquid 2 before the exchange of gas(es) molecules between the gas flow 8 and the liquid flow 9. Then, the sensor(s) 3 transmits the data to the control module 13 (not shown in **Figure 1****,** see **Figure 3**) for example, before the liquid 2 contacts the gas-permeable membrane 4.

It is then possible to measure the concentration of dissolved gas(es) and/or the pH of the liquid 2 before the gas-permeable membrane 4, and then after it contacts the gas-permeable membrane 4. Thus, it is possible to verify the changes on dissolved gas(es) concentration and/or pH produced in the liquid 2 when a specific gas, or gas mixture, is injected by the gas controller with the desired composition (i.e nature of the gas(es) and concentration or percentage of each gas in the total gas mixture flow 8) and/or with the desired gas and/or liquid flow rate. Advantageously, when the microfluidic channel B1 comprises at least two dissolved gas(es) concentration and/or pH sensors 3 and 5 (one before and one after the gas permeable-membrane 4) it is possible to better control and adjust: pressure(s) or partial pressure(s), gas and/or liquid injection flow rate, gas(es) composition (i.e nature of the gas(es) and concentration and/or percentage of each gas in the total gas mixture flow 8).

In this embodiment, the liquid flow 9 circulates through the microfluidic channel B1 and crosses the overlapping zone 30 with the microfluidic channel A1 at the gas-permeable membrane 4 simultaneously with the gas(es) flow 8. The liquid 2 is thus exchanging molecules with gas(es) in a gas(es) exchanging zone Z-EXCH thanks to the diffusion and local pressure principle (symbolized by the black arrows). For example, the exchange can consist of gas(es) enrichment, i.e. gas(es) molecules diffuse into the liquid 2. In another example, the exchange may consist in depleting the liquid of gas(es), as is the case when hypoxia is induced by depleting oxygen.

The gas(es) containing liquid continues to flow in the microfluidic channel B1 until it comes into contact with a biological entity 7 cultured in the channel B1. The microfluidic channel B1 in which the biological entity 7 is cultivated is then considered as a closed chamber, i.e., a hermetically sealed environment, whose parameters can be controlled without being in direct contact with the outside environment by no other means than the gas, or gas mixture, 1 or the liquid 2. Thus, the microfluidic device A according to **Figure 1** (for example a microfluidic chip) allows to cultivate biological entities 7 for the long-term cultivation, where the long term lasts between 1 day and 60 days, in a closed hermetic environment, while limiting external contamination, and controlling dissolved gas(es) composition and/or pH of the cell culture medium.

The dissolved gas(es) concentration and/or pH are measured thanks to the dissolved gas(es) concentration and/or pH sensor(s) 5 placed in the microfluidic channel B1, only after or after and before, the gas-permeable membrane 4. As previously described, this sensor(s) 3, 5 is configured to collect the dissolved gas(es) concentration and/or pH measurements and, for example, transmit the data to the control module 13 (not shown in **Figure 1****,** see **Figure 3**)**.**

This control module 13 can be configured to receive the data collected by the dissolved gas(es) concentration and/or pH sensor(s) 5.

In a particular embodiment, when the microfluidic device A comprises a second dissolved gas(es) concentration and/or pH sensor(s) 3, placed before the gas-permeable membrane 4, as described previously, the control module 13 can be also configured to receive the data collected from this second sensor 3.

In an embodiment, the control module 13 is configured to display on a human-machine interface the data collected by the dissolved gas(es) concentration and/or pH sensor(s) 5 and the dissolved gas(es) concentration and/or pH sensor(s) 3 when the microfluidic device A has one. The operator then analyzes and manually modifies fluid flows (i.e., liquid and/or gas(es) flow rate and/or pressure, and/or gas(es) composition) *via* the various flow controllers (gas flow controller and liquid flow controller) to obtain a specific gas(es) composition and/or pH value in the liquid cell culture medium 2. This analysis involves comparing the data received from the various sensors 3, 5 with a predetermined gas(es) composition and/or pH of the liquid cell culture medium 2, as well as taking into account fluid flow rates to be less or equal to 50ml/min and local pressures to be less or equal to 100 bar within one of both fluidic circuits. The operator then modifies (or adjusts) manually the gas(es) flow rate and/or pressure and/or the liquid flow rate and/or pressure via the different flow controllers.

Alternatively, in another embodiment, the control module 13 is also configured to analyze and thus compare the collected dissolved gas(es) concentration and/or pH measurements to a predetermined dissolved gas(es) composition and/or pH value of the flowing liquid 2.

It should be noted that by adjusting CO₂ concentration of the cell culture medium, its pH can be determined. Indeed, for any given partial pressure of a gas, solubility is inversely proportional to temperature in aqueous solutions and decreases at increased concentration of electrolytes. These can modify indirectly other parameters, for example, when CO₂ dissolves in water it creates carbonic acid (H₂CO₃) and the hydrogen ions present in carbonic acid make water acidic thus lowering the pH.

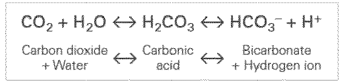

In the embodiment in which the microfluidic device A comprises a second dissolved gas(es) concentration and/or pH sensor(s) 3, placed before the gas-permeable membrane 4, the control module 13 is then able to compare the dissolved gas(es) concentration and/or the pH value collected by the first sensor 5 (i.e, the sensor placed after the gas-permeable membrane 4), with the dissolved gas(es) concentration and/or the pH value collected by the second sensor 3 (i.e, the sensor placed before the gas-permeable membrane 4). Then these data are compared to a predetermined dissolved gas(es) composition and/or pH value (s) of the flowing liquid 2.

By comparing the data collected by the sensor 5 after the gas-permeable membrane 4 with a predetermined dissolved gas(es) composition and/or pH value(s), or by comparing the data collected by the two sensors 3 and 5 (before and after the gas-permeable membrane 4) with the predetermined gas(es) composition and/or pH value(s), the control module 13 is able to adjust the different parameters (i.e. pressure(s) or partial pressure(s), gas and/or liquid injection flow rate(s), gas composition (i.e nature of the gas(es) and concentration and/or percentage of each gas in the total gas mixture flow 8)) by transmitting adjustment commands to the gas and/or liquid controller to obtain a specific gas(es) composition and/or pH value in the liquid cell culture medium 2.

For example, the control module 13 is able to adjust the gas flows given a fixed liquid flow by transmitting adjustment commands to the gas controller to obtain a specific gas(es) composition and/or pH value in the liquid cell culture medium 2.

Alternatively, the control module 13 process the data as previously described, but only display the result on a human-machine interface and the operator manually adjust the fluid flow rate and/or pressure accordingly *via* the different fluids controller.

Thus, the adjustment is done to adjust the gas molecules transfer between the gas and the liquid through the gas-permeable membrane using the dissolved gas(es) concentration and/or pH sensor(s) to monitor these values and adjust the liquid and/or gas(es) flow rate and/or pressure(s) accordingly to achieve the desired dissolved gas(es) composition and/or pH value(s).

Furthermore, the control module 13 is configured to adjust the gas(es) composition and/or pH of the liquid cell culture medium while maintaining a fluid flow rates to be less or equal to 50ml/min and local pressures to be less or equal to 100 bar within at least one of both fluidic circuits.

More particularly, in order to obtain a desired dissolved gas(es) composition and/or pH of the liquid 2 flowing at a predetermined flow rate, i.e suitable for the culture of the biological entity 7, the control module 13 is configured to transmit a command of adjustment of the gas(es) injection flow rate and/or gas(es) pressure to the gas flow controller. In particular, when the gas is a gas mixture, the control module 13 is also configured to transmit a command of adjustment of the percentage or concentration of each gas in the total gas flow 8 and/or the partial pressure of each gas, to the gas flow controller.

Alternatively, or in addition, the control module 13 is also configured to transmit a command of adjustment of the liquid injection flow rate and/or liquid pressure to the liquid flow controller in order to achieve the predetermine gas(es) composition and/or pH while maintaining a fluid flow rates to be less or equal to 50ml/min and local pressures to be less or equal to 100 bar within at least one of both fluidic circuits.

For this, the control module 13 implements a mathematical algorithm (e.g., proportional feedback closed-loop control, active disturbance rejection control (ADRC)) and/or an artificial intelligence module (e.g., neural network proportional-integral-derivative control) that enable to compare the collected data to a predetermined dissolved gas(es) composition and/or pH value(s) in order to adjust gas or gas mixes flows, while maintaining fluid flow rates to be less or equal to 50 ml/min and local pressures to be less or equal to 100 bar within at least one of both microfluidic circuits, to obtain stable predetermined dissolved gas(es) composition and/or pH values. Then, using a feedback loop based on the measured dissolved gas concentration and/or pH of said liquid cell culture medium 2 and fluid flow rates to be less or equal to 50ml/min and local pressures to be less or equal to 100 bar within at least one of both fluidic circuits, the control module 13 is able to transmit the commands of adjustment described above to meet the predetermine dissolved gas(es) composition and/or pH of the liquid cell culture medium.

Thus, it is possible to achieve the right dissolved gas(es) composition and/or pH for a desired liquid flow 9 that has been set to guarantee in time: the dissolved gas(es) composition, the pH and the shear stress need to cultivate the biological entity 7.

In an embodiment, to further analyze the biological entity(ies) 7, the microfluidic device A allows the recovery of the biological entity(ies) 7 at the end of its operation for further analysis using complex or bulky techniques such as for example staining, advanced microscopy, or DNA analysis.

Furthermore, in another embodiment, instead of going to a waste container, a volume of liquid cell culture medium 2 is flown outside the microfluidic device A and is retrieved (for example using a recovery container) for further processing and analysis using common molecular and cellular techniques.

Alternatively, or in addition, a volume of liquid cell culture medium 2 is recuperated inside the microfluidic device A to further analyze important aspects of the experiment and perform complex analysis such as for example, but not limited to, to determine the presence or quantify metabolites, cell-free DNA (cfDNA), circulating cells, RNA or proteins. For this, a specific microfluidic channel is added before the fluid outlet 10 to the second microfluidic circuit (i.e dedicated to the circulation of the liquid 2) in order to direct a certain quantity of liquid 2 to a recovery container or to an analysis device.

Advantageously, the channel B1, in which the liquid flow 9 circulates, comprises sensor(s) 6 configured to measure and/or identify physicochemical parameter(s) of the liquid 2 such as: temperature, metabolites (e.g., lactate, lipids, organic acids, bilirubin), nutrients (e.g, amino acids, carbohydrates), proteins (e.g., albumin, protein C, protein S), nucleic acids (e.g., Deoxyribonucleic acid (DNA), ribonucleic acid (RNA)) ... etc.

This is particularly advantageous since the liquid cell culture medium 2 stay isolated from the outside environment, reducing contamination risk. It should be noted that these additional sensors 6, can be additionally made compatible with recovery of a volume of liquid 2 either outside the microfluidic device A, or directly inside as described above.

In a variant, another microfluidic circuit designed to be independent and not connected to the other is added. A thermalization solution, namely a liquid capable of transferring heat, is circulated to control the temperature of the microfluidic device A. An external device for automatically heating or cooling the liquid and pumping mechanisms is then necessary for real-time temperature control.

After having circulated in their assigned channels A1 or B1, the gas(es) flow 8, and the liquid flow 9 are evacuated from the microfluidic device A through a fluid outlet(s) 10 to a waste container (not shown) or in some embodiment to a recovery container and /or analysis device as described previously.

It should be noted that we are not limited to the configuration described above. Indeed, in other embodiments, it is possible to have a plurality of gas-permeable membrane 4 with a plurality of dissolved gas(es) concentration and/or pH sensor(s) 3 and 5. In other embodiment, it is possible to have a plurality of gas-permeable membrane 4' and a plurality of dissolved gas(es) concentration and/or pH sensor(s) 3' and 5'. In other words, we can have as many as needed gas-permeable membrane and sensors, in different configuration. The same apply for the following embodiment described in relation to figures 2 to 5.

We described now in relation with **Figure 2** an example of an embodiment of a fluidic device or fluidic assembly A suitable for implementing the method according to the invention. The method according to the invention will be described in relation to **Figure 3** below.

The fluidic device D or fluidic assembly A according to **Figure 2** comprises at least the same features as described in relation to **Figure 1****,** which have at least the same advantages as those described in relation with **Figure 1****.**

However, in these embodiments, the biological entity(ies) 7 is cultured in a single reservoir 14, not in the microfluidic channel B1.

In one embodiment, the reservoir is part of the fluidic device D. In other words, the reservoir could be integrated in the microfluidic channels without being an external additional element, just forming an integrated device with microchannels flowing into wells. For example, without nozzles, just a channel in series with a reservoir, entering in one side and exiting from the other.

In another embodiment, this reservoir is completely sealed by a microfluidic device D, and thus forming a fluidic assembly A to constitute the closed chamber 14. The reservoir can be an isolated reservoir or a well that, in some embodiments, could be part of a multi-well cell culture plate as described in relation with **Figures 4A** **and** **5** below.

Thus, when the reservoir(s) is(are) integrated in a microfluidic device, thus it form the fluidic device D. When the microfluidic device D is mounted on reservoir(s), it forms the fluidic assembly A.

It should be noted that, it is possible to have several reservoirs mounted in series and/or in parallel to each other. In other words, the microfluidic device D according to **Figure 2****,** **4A** **and** **5** is configured to integrate one or more reservoirs or to adapt to one or more reservoirs thus forming one or more closed chambers 14 and 15 mounted in series and/or in parallel and in which it is possible to cultivate biological entities 7.

It should be noted that the biological entity(ies) 7 cultured in the first closed chamber 14 can be identical or different from the one culture in the second closed chamber 15.

These closed chambers 14, 15 comprise a liquid volume VL and a gas volume VG.

In order to supply the closed chamber 14 with liquid 2, i.e., cell culture medium 2, the microfluidic channel B1 forming the first microfluidic circuit is discontinuous. Unlike the microfluidic channel A1, which carries the gas or gas mixture, which is continuous.

In other words, the channel B1 enters at the top of the closed chamber 14 and extends to a certain height in the liquid volume, thus forming a liquid inlet IN. Because the liquid flow 9 is continuous, and the gas volume VG in the closed chamber 14 is constant, the liquid 2 exits the closed chamber 14 *via* a liquid outlet OUT formed by channel B1 that goes out of the closed chambers 14 from a certain height of the bottom of the closed chamber 14 to the top of the closed chamber 14.

The liquid inlet IN and outlet OUT formed by the microfluidic channel B1 can be in the form of nozzle or orifice or duct as described in relation with **Figure 5****.**

When the microfluidic device D integrate a single isolated reservoir or is mounted on a single isolated reservoir, the liquid drains to a fluid outlet 10 as previously described in relation to **Figure 1****.**

Alternatively, as shown in relation with **Figure 2****,** the channel B1 continues and arrives in another closed chamber 15. The liquid 2 from the first closed chamber 14 flows through the channel B1 to a second closed chamber 15. Then, as described above, the liquid 2 flows out of the second closed chamber 15 to a fluid outlet 10 as previously described in relation to **Figure 1****.**

Thus, it should be noted that in the case of several closed chambers we are not limited to two closed chambers. Indeed, just as with two closed chambers, it is possible to circulate the liquid in several other chambers that could be in series or/and in parallel through bifurcating microfluidic channels.

Advantageously, before entering the second closed chamber 15, the microfluidic channel B1 and the microfluidic channel A1 can communicate through a second gas-permeable membrane 4'. The microfluidic channel in which the liquid 2 flows comprises a dissolved gas(es) concentration and/or pH sensor 5' after the gas-permeable membrane 4'. In variant, the microfluidic channel B1 also comprises a second dissolved gas(es) concentration and/or pH sensor(s) 3' placed before the gas-permeable membrane 4'. Thus, it is possible to modify the dissolved gas(es) composition and/or the pH in the liquid 2 coming out of the first closed chamber 14 before entering the second closed chamber 15 at the level of a gas exchanging zone Z-EXCH.

In the same way as described above in relation to **Figure 1****,** the data collected by the dissolved gas(es) concentration and/or pH sensor(s), after or before and after the gas permeable membrane 4, 4' before entry into a closed chamber 14, 15, can be transmitted to the control module 13 for analysis and adjustment of the flows if necessary.

Advantageously, the fluidic device D or fluidic assembly A also comprises a dissolved gas(es) concentration and/or pH sensor 12 after the last closed chamber just before the flow outlet 10. This allows for example to monitor the effect of the cell or biological entity culture on the media dissolved gases by comparison of the quantified values of sensor(s) 12 with the quantified values of sensor(s) 5' due to consumption or metabolization processes.

The method for controlling fluid displacement and gas(es) composition and/or pH of a liquid, such as a liquid cell culture medium, according to the invention, and implemented by a microfluidic device A, fluidic device D or fluidic assembly A described in relation with **Figures 1, 2****,** or **5****,** is now described in connection with **Figure 3****.**

In this example, the method described in relation with **Figure 3** is implemented by the fluidic device D or the fluidic assembly A described in relation with **Figure 2****.** However, it should be noted that the microfluidic devices described in relation with **Figure 1** and fluidic device A or fluidic assembly A described in relation to **Figure 2** **and** **5** are equally suitable for implementing the method described here.

In order to simplify, we called device (A, D) the microfluidic device A according to **Figure 1****,** the microfluidic device D, the fluidic device A or the fluidic assembly A according to **Figures 2** **and** **5****.**

The method for controlling fluid displacement and gas(es) composition and/or pH of a liquid, such as a cell culture medium according to the invention comprises:
- a step of injecting a gas, or gas mixture 1 by at least one gas flow controller (or gas controller) (not shown) into one or more fluid connection orifice(s) or fluidic connection duct(s) (not shown) allowing the attachment of at least one fluid connector (not shown) coming from the gas flow controller, then through at least one first microfluidic circuit of the device (A, D). The injected gas flow 8 then circulated in at least one microfluidic channel A1 towards a fluid outlet 10. Alternatively, or in addition a sequence of different gas can be injected;
- a step of injecting the liquid cell culture medium 2 by at least one liquid flow controller (or liquid controller) (not shown) into one or more fluid connection orifice(s) or fluidic connection duct(s) (not shown) allowing the attachment of at least one fluid connector (not shown) coming from the liquid flow controller, then through at least one second microfluidic circuit of the device (A, D). Alternatively, or in addition a sequence of different liquid can be injected. The injected liquid flow 9 then circulates through at least one microfluidic channel B1 toward at least one closed chamber 14 in which a biological entity 7 is cultured;
- a step of producing a diffusion and pressure driven exchange of gas, or gas mixtures 1, between said liquid cell culture medium 2 and a volume of gas, or gas mixture 1 circulating in the at least one first microfluidic circuit, through at least one gas-permeable membrane 4 forming the overlapping zone 30 between said at least one first microfluidic circuit in which the gas, or gas mixture is flowing and said at least one second microfluidic circuit in which the liquid cell culture medium 2 is flowing;
- a step of measuring a dissolved gas(es) concentration and/or pH of said liquid cell culture medium 2 by at least one dissolved gas(es) concentration and/or pH sensor 5 in contact with the liquid 2 and embedded inside said at least one second microfluidic circuit and transmitting said measurements to a control module 13;
- a step of adjusting the injection flow rate and/or pressure of said gas, or gas mixture, 1 by said at least one gas controller and/or the injection flow rate and/or pressure of said liquid cell culture medium 2 by said at least one liquid controller, based on said measured dissolved gas(es) concentration and/or pH of said liquid cell culture medium (2), fluid flow rates to be less or equal to 50ml/min and local pressures to be less or equal to 100 bar within at least one of both fluidic circuits and a predetermined dissolved gas, or gas mixture 1, composition and/or pH of the liquid cell culture medium (2).

The method according to the invention also comprises a step of determining the injection flow rate and/or pressure of said gas, or gas mixture 1 to be reached and/or the injection flow rate and/or pressure of said liquid cell culture medium 2 to be reached based on said dissolved gas(es) concentration and/or pH of said liquid cell culture medium measurements, to obtain a fluid flow rates to be less or equal to 50ml/min, local pressures to be less or equal to 100 bar within at least one of both fluidic circuits and a predetermined dissolved gas, or gas mixture, composition and/or pH value. The method also comprises a step of transmitting a command to adjust the injection flow rate and/or pressure of said gas, or gas mixture 1 to the gas controller and/or the injection flow rate and/or pressure of said liquid cell culture medium 2 to the liquid controller.

As previously described in relation with **Figure 1****,** this step of determining and transmitting a command is done "manually" by an operator or "automatically" by the control module 13.

Advantageously, the step of measuring the dissolved gas(es) concentration and/or pH of said liquid cell culture medium 2 is done after the at least one gas-permeable membrane 4. In other words, the dissolved gas(es) concentration and/or pH of said liquid cell culture medium 2 measures are taken after the gas(es) flow and liquid flow have exchange molecule(s).

In a particular embodiment, the step of measuring the dissolved gas(es) concentration and/or pH of said liquid cell culture medium 2 is done before and after the at least one gas-permeable membrane 4. In other words, the dissolved gas(es) concentration and/or pH of said liquid cell culture medium 2 measures are taken before and after the gas(es) flow and liquid flow have exchanged molecule(s).

In a particular embodiment, the step of measuring the dissolved gas(es) concentration and/or pH of said liquid cell culture medium 2 is done after the at least one closed chamber 14, 15. In other words, according to this particular embodiment, it is possible to measure the dissolved gas(es) concentration and/or pH of the liquid cell culture medium 2 when it comes out of a closed chamber in which a biological entity(ies) 7 is cultivated. This is particularly advantageous, when several closed chambers are mounted in series and/or in parallel one after the other (as shown in **Figures 2, 3** **or** **5**). Thus, it is possible to know how the dissolved gas(es) concentration and/or pH has evolved after the liquid 2 has been in contact with a biological entity(ies) 7 in a closed chamber.

In some embodiments, when the liquid is destined to flow into another closed chamber, it is then possible to adjust the dissolved gas(es) composition and/or pH if necessary, before entering this other closed chamber. For this purpose, another gas-permeable membrane 4' is located after the dissolved gas(es) concentration and/or pH sensor and before the other closed chamber to adjust the gas(es) composition and/or pH of the liquid cell culture medium 2. This method is applicable to a plurality of serial and/or parallel closed chambers communicated by microfluidic channels.

As described previously in relation with **Figures 1, 2****,** in some embodiments the control module 13 is configured to receive the data collected by the dissolved gas(es) concentration and/or pH sensor(s) and to process it. The control module 13 then transmit the command(s) to adjust the different parameters (i.e. pressure(s) or partial pressure(s), gas and/or liquid injection flow rate (s), gas composition (i.e nature of the gas(es) and concentration and/or percentage of each gas in the total gas mixture flow 8)) to the gas flow controller and/or the liquid flow controller.

The term "module" may correspond to a software component as well as to a hardware component or a set of hardware and software components, a software component corresponding itself to one or more computer program(s) or subprogram(s) or more generally to any element of a program able to implement a function or a set of functions.

In one embodiment, the control module 13 is independent from the liquid flow controller or the gas flow controller.

In another embodiment the control flow module 13 in embedded in the gas flow controller and/or the liquid flow controller.

According to a particular embodiment of the invention, the step of determining the dissolved gas(es) concentration and/or pH of the liquid cell culture medium 2 by the control module 13 comprises determining the fluid (i.e gas, gas mixture, or liquid) injection flow rates and/or pressures to be applied by the gas flow controller and/or liquid flow controller depending on the measured dissolved gas(es) concentration and/or pH of the liquid cell culture medium 2 to obtain a predetermined dissolved gas, or gas mixture 1, composition and/or pH of the liquid cell culture medium 2 while maintaining the fluid flow rates to be less or equal to 50ml/min and local pressures to be less or equal to 100 bar within at least one of both fluidic circuits.

Indeed, in an example the fluid (i.e. gas and liquid) flow rates are set to be less or equal to 50ml/min and local pressures of the gas and liquid are set to be less or equal to 100 bar within both microfluidic circuits. Preferably, liquid injection flow rates and/or pressures will be set first to control the shear stress applied to the cell or biological sample culture and the gas or gas mixture injection flow rates and/or pressures adjusted to attain the expected dissolved gas(es) composition and/or pH in the cell culture medium for the cell or biological sample culture 7.

This determination can be made by implementation of a mathematical algorithm (e.g., proportional feedback closed-loop control, active disturbance rejection control (ADRC)) and/or an artificial intelligence module (e.g., neural network proportional-integral-derivative control) configured to achieve automatically the desired dissolved gas(es) composition and/or pH values by using the data received by the control module 13 from the dissolved gas(es) concentration and/or pH sensor(s) to adjust accordingly fluid flow rates, gas mixture proportions, and/or local pressures within one or within both microfluidic circuits.

The method according to the invention further comprises a step of successive displacement of volume of liquid cell culture medium VL from at least one first chamber 14 to at least one second chamber 15 through said at least on second microfluidic circuit of the device D when several closed chambers 14, 15 are mounted in series and/or in parallel with each other.

In a particular embodiment, the method according to the invention further comprises: a step of measuring and/or identifying, by at least one sensor 6 embedded inside said at least one second microfluidic circuit in which the liquid cell culture medium 2 is flowing, at least one physicochemical parameter of the liquid cell culture medium 2. This physicochemical parameter being chosen from, but not limited to:
- temperature,
- metabolite(s),
- nutrient(s),
- protein(s),
- nucleic acid(s).

We now show, in relation with **Figures 4A** and **5** another example of a structure of an embodiment of the fluidic assembly A according to the invention, which is suitable for implementing the method according to the invention previously described.

It should be noted that the features described in relation to **Figure 2** can be applied to **Figure 5****.**

A microfluidic device D with shape selected from among the cubic, rectangular shapes but preferably substantially parallelepiped-shaped comprises a cover, also called fluid-routing layer 25, and a part called the liquid-routing layer 21 (also called manifold 21), comprising a series of nozzles or orifices or ducts 214 forming the base of this microfluidic device D. The nozzles or orifices or ducts 214 carry the liquid into the multi-well cell culture plate 20 and out of it, as described in **Figure 2****.**

The fluids-routing layer 25 has, on two opposite edges, one or more connection orifice(s) or fluidic connection duct(s) 251, 255 for attaching at least one fluid connector (not shown) from a gas flow controller or a liquid flow controller (not shown). As previously described, the gas or liquid flow controller externally supplies a fluid such as a gas, gas mixture or a liquid to the microfluidic device D according to the invention. The gas or liquid flow controller controls in real time the fluid pressure and/or of its flow rate (or injection flow rate). This fluids-routing layer 25 can be entirely opaque or, in a variant, allow the passage of light in order to obtain a real-time optical image of the biological entities.

In this variant, on the external top surface of the fluids-routing layer 25, several zones, also called apertures 253 allow the passage of light and thus a visualization by microscope or by other means necessary to obtain real-time images of the biological entities.

The fluids-routing layer 25 preferably has a flat surface at least on its bottom face. It can be manufactured with different materials including, but not limited to, polymers such as PMMA (poly(methyl methacrylate)), cyclo olefin polymer/copolymer (COP/COC), PS (polystyrene), PEEK (polyetheretherketone) or PC (polycarbonate).

A transparent layer 24 is then attached to the flat surface of the bottom part of fluids-routing layer 25. This transparent layer 24 allows the selective sealing of the microfluidic device D and structures and light conduction to allow or facilitate microscopy-based imaging. This transparent layer 24 comprises several holes 241, 245 allowing the passage of the fluid from the top layer, i.e the fluids-routing layer 25 to the following layers. More particularly, some holes 241 are dedicated to the transport of liquid, such as cell culture medium, and other holes 245 are dedicated to the transport of gas(es).

In a variant, this transparent layer 24 could be opaque by just working as a sealer of the fluidic chambers in the assembly A.

In order to align all the different layers of the microfluidic device D, i.e the fluids-routing layer 25, the transparent layer 24, the gas-routing layer 23, the gas-permeable membrane 22, the liquid-routing layer 21, the transparent layer 24 also comprises several aligning motives 242.

The gas-routing layer 23, is then attached between the bottom surface of the transparent layer 24 and the top surface of the gas-permeable membrane 22.

This gas-routing layer 23 comprises several holes 231 allowing the passage of the liquid from the top layers to the liquid-routing layer 21.

This gas-routing layer 23 comprises one or several microfluidic channels 235 that are able to carry a gas or gas mixture. More particularly, the gas or gas mixture enters the microfluidic device D to the corresponding connection orifice(s) or fluidic connection duct(s) 255 of the fluids-routing layer 25, then circulates through one or more corresponding hole 245 of the transparent layer 24, then to the microfluidic channel(s) 235 of the gas-routing layer 23 connects.

In order to align the gas-routing layer with the other layer of the microfluidic device D, it comprises aligning motives 232. The gas-routing layer 23 may also comprise apertures 233 to conduct light and allow or facilitate microscopy-based imaging.

The gas-permeable membrane 22 is then attached between the bottom surface of the gas-routing layer 23 and the top surface of the liquid-routing layer 21. This gas-permeable membrane 22 comprises several holes 221 allowing the passage of the liquid from the top layers to the liquid-routing layer 21.

Like the upper layers (i.e transparent layer 24, gas-routing layer 23), the gas-permeable membrane 22 comprises aligning motives 222 for its alignment with all the layers of the microfluidic device D and, in a variant, several apertures 223 to conduct light to allow or facilitate microscopy-based imaging.

The liquid-routing layer 21, also called manifold 21, is attached to the bottom surface of the gas-permeable membrane 22 and forms the base of the microfluidic device D according to **Figure 4A** and **5****.** More particularly, the manifold 21 is configured to be adaptable on the top of a multi-well cell culture plate 20.

This liquid-routing layer 21 comprises one or several microfluidic channels 211 able to carry liquid, such as cell culture medium.

In order to carry the liquid to one or more well of the multi-well cell culture plate 20, the liquid-routing layer 21 comprises nozzles or orifices or ducts 214 carrying the liquid into the well(s) of the multi-well cell culture plate 20 and out of it. In other words, at least a pair of nozzles or orifices or ducts 214 goes into one well of the multi-well culture plate 20 in order to for a liquid inlet and a liquid outlet (as described in relation with **Figure 2**). More particularly, the liquid enters the microfluidic device D to the corresponding connection orifice(s) or fluidic connection duct(s) 251 of the fluids-routing layer 25 and then passes through one or more holes 241 of the transparent layer 24, and then to the microfluidic channel(s) 211 of the liquid-routing layer 21, then to the nozzle(s) or orifice(s) or duct(s) 214 which ends up in the different wells of the multi-well culture plate 20.

The liquid-routing layer, or manifold 21 is a structure defining microfluidic nozzle(s) or orifice(s) or duct(s) 214 and a cavity or empty space 215 reaching a defined depth in the well(s) 34, 35 of the multi-well culture plate 20 while sealing a microfluidic circuit dedicated to the circulation/transport of liquid, such as cell culture medium. The cavity or empty space 215 can for example be substantially cylindrical, parallelepiped or cube shaped.

This microfluidic circuit for liquid is defined by:
- the connection orifice(s) or fluidic connection duct(s) 251 of the fluids-routing layer 25,
- the corresponding hole(s) 241 of the transparent layer 24,
- the microfluidic channel(s) 211 of the liquid-routing layer 21,
- the nozzle(s) or orifice(s) or duct(s) 214 of the liquid-routing layer 21.

The nozzle(s) or orifice(s) or duct(s) 214 and the cavity or empty space 215 reach a defined depth in the multi-well culture plate 20, while sealing the microfluidic circuit for liquid.

In a variant, the microfluidic device D comprises one or more microfluidic nozzles or orifices or ducts with different heights to control (in combination with the given gas pressures and/or flow rates) which liquid is perfused in the closed chamber(s) which contain the biological entity. These microfluidic nozzles or orifices or ducts are designed to control, over time, the selective application of a given dose of specific compounds such as drugs or toxic particles mixing with the usual life-sustaining mediums for the biological entity.

The liquid-routine layer 21 allows the microfluidic device D to be adapted on the top of a multi-well cell culture plate 20. In other words, it facilitates the assembly of the device D to the wells of the multi-well culture plate 20 and guarantees the sealing of the resulting structure, said fluidic assembly A.

Like the upper layers (i.e transparent layer 24, gas-routing layer 23, gas-permeable membrane 22), the liquid-routing layer 21 comprises aligning motives 212 for its alignment with all the layers of the microfluidic device D and optionally several apertures 213 to conduct light and allow or facilitate microscopy-based imaging.

This sandwich structure can apply to the the microfluidic device D of the fluidic assembly A according to **Figure 4A** to **5****,** and its variants, are embodied to guarantee the correct assembly of the different layers into a microfluidic structure allowing: selective communication with a gas and/or liquid flow controller while avoiding undesirable contamination.

In a variant of this structure, additional microfluidic, membrane or transparent layers can be added to form more complex and/or parallel 3D fluidic structures exploiting the vertical dimension. In another variant, the different layers can be integrated into single bodies by the use of different manufacturing techniques such as injection molding, 3D printing, or thermal bonding, but not only.

In particular, it should be noted that the 3D construction of the microfluidic device D, fluidic device A or fluidic assembly A could be fabricated or assembled by other means and several or all of the layers could fuse together in any manner allowing to keep the functionality and 3D structure of the fluidic device D or fluidic assembly A.

Other microfluidic layers could be also added to accomplish different objectives (e.g. 3D microfluidic structures; multiple liquids, gases or gases mixes flows parallelized in different layers) and functionalities (e.g. temperature control by flowing an independent thermalization solution and using temperature sensors).
For example, in an embodiment according to the invention, one or more thermalisation fluid(s) with externally controlled temperature can be sent into different or similar selected parallel microfluidic channels in order to regulate the temperature of all the wells of the multi-well plate. In another variation, the temperature of certain selected wells only is regulated by the injection of one or more thermalisation liquid(s) into selected microfluidic channels. In another variant, specific fluids are integrated to perfuse the thermosetting solutes and control the temperature(s) of the device or of certain parts of the device.

In particular, the assembly of the microfluidic device according to **Figures 4A** and **4B** on a multi-well plate is shown in **Figure 5** forming the fluidic assembly A.

The microfluidic device D is assembled to the wells 34, 35 of the multi-well culture plate 20. This assembly guarantees the sealing of the resulting cell culture system, for example by means of one or more O-ring seals or equivalent structure, or even by bonding. More specifically, the microfluidic device D is mounted on the top part of a standard multi-well culture plate 20, as subsequently shown in relation to **Figures 4A** **and** **5****,** and compressed by external means, so that the cell culture fluidic assembly A according to the invention is completely sealed, except for the connecting orifice(s) or the fluidic connecting duct(s) 251, 255. Thus, when the microfluidic device D is mounted on a multi-well cell culture plate the association of the well(s) 34, 35 of the multi-well plate 20 with the bottom part of the microfluidic device D, i.e the liquid-routing layer 21, forms one or several closed chamber, mounted in series and/or in parallel equivalently to **Figure 2****.**

Before this assembly, the biological entities are placed in the multi-well plate 20, along with the reagents, before closing it with the microfluidic device D. The mediums, samples, cell cultures and reagents are placed in the wells 34, 35 of the multi-well plate 20 before the experiment (as is currently the case in pharmaceutical, biotechnological and biological laboratories) and remain isolated throughout its use after the assembly of the fluidic assembly A according to the invention.

When assembled, the two parts (i.e., microfluidic device D and multi-well culture plate 20) are sealed and connected to the exterior only by the connecting orifice(s) or by fluidic connecting duct(s) 251, 255. The reagents and the biological entities are completely isolated from the exterior since the orifice(s) 251, 255 are used to circulate fluids: either a gas, or a gas mixture or a liquid (which are preferably prefiltered to prevent contamination). When using thermalization solutions, the thermalization solutions flow in independent microfluidic circuits in order to never come in contact with the reagents or the biological samples.

This fluidic assembly A, ie. microfluidic device D and multi-well culture plate 20, is then connected to one or more fluid controllers: liquid flow controller(s) or gas flow controller(s). It should be noted that the connecting orifice(s) or the fluidic connecting duct(s) 251, 255, and thus by extension the fluidic assembly A, can be connected to one or more fluid controllers. Alternatively, a fluid controller can be connected to several connecting orifice(s) or fluidic connecting duct(s) 251, 255. However, either a gas, or mixture of gases, or a liquid is injected into connecting orifice(s) or fluidic connecting duct(s) 251, 255.

Thus, the cell culture system according to the invention, suitable to implement the method according to the invention described previously in relation to **Figure 3****,** comprises:
- a microfluidic device A according to **Figure 1** or a fluidic device A or assembly A according to **Figures 2 to 5****;**
- a manual or computer-controlled gas, or gas mixture, flow controller configured to control the pressure and/or injection flow rate in real-time;
- a manual or computer-controlled liquid flow controller configured to control the pressure and/or injection flow rate in real time;
- an external gas, or gas mixture, and liquid supply (not shown),
- the control module described previously in relation with **Figures 1** to **3****,**
- one or more connector(s) for the application of gas(es) or liquid.

The different embodiments described previously in relation with **Figures 1** to **5** can be combined with each other and have the same advantages. In particular, the different embodiments of the microfluidic device A or the fluidic device A or fluidic assembly A all enable the method according to the invention to be implemented when integrated into a cell culture system (also called microfluidic cell culture system) as described above.

## Claims

1. A method for controlling fluid displacement and dissolved gas(es) composition and/or pH of a volume of liquid cell culture medium (VL) contained in at least one closed chamber (14, 15), **characterized in that** the method comprises:
- an injection of a gas, or gas mixture (1) by at least one gas controller,
- an injection of a liquid cell culture medium (2) by at least one liquid controller into said at least one closed chamber (14, 15),
- a production of a diffusion and pressure driven exchange of gas, or gas mixtures (1), between said injected liquid cell culture medium (2) and said injected gas, or gas mixture (1) through at least one gas-permeable membrane (4, 22),
- a collection of measurements of a dissolved gas(es) concentration and/or pH of said liquid cell culture medium (2) by at least one dissolved gas(es) concentration and/or pH sensor (5),
- a determination of an injection flow rate and/or pressure of said gas, or gas mixture (1), and/or gas mixture composition, and/or an injection flow rate and/or pressure of said liquid cell culture medium (2) based on said measured dissolved gas(es) concentration and/or pH of said liquid cell culture medium (2), fluid flow rate to be less or equal to 50ml/min, local pressures to be less or equal to 100 bar and a predetermined dissolved gas, or gas mixture (1), composition and/or pH of the liquid cell culture medium (2),
- an adjustment of an injection flow rate and/or pressure of said gas, or gas mixture (1) by said at least one gas controller and/or an injection flow rate and/or pressure of said liquid cell culture medium (2) by said at least one liquid controller based on said determination to obtain said predetermined dissolved gas, or gas mixture (1) composition and/or pH of the liquid cell culture medium (2).

2. The method according to claim 1, **characterized in that** said collection of measurements of the dissolved gas(es) concentration and/or pH of said liquid cell culture medium (2) is done after the production of the diffusion and pressure driven exchange of gas, or gas mixtures (1), between said liquid cell culture medium (2) and the gas, or gas mixture (1) through said at least one gas-permeable membrane (4, 22).

3. The method according to claim 2, **characterized in that** said method further comprises a collection of measurements of dissolved gas(es) concentration and/or pH of said liquid cell culture medium before the production of the diffusion and pressure driven exchange of gas, or gas mixtures (1), between said liquid cell culture medium (2) and the gas, or gas mixture (1) through said at least one gas-permeable membrane (4, 22).

4. The method according to claim 1 to 3, **characterized in that** said method further comprises a collection of measurements of the dissolved gas(es) concentration and/or pH of said liquid cell culture medium after said liquid cell culture medium has passed through at least one biological entity (7) cultured in the at least one closed chamber (14, 15).

5. The method according to claim 1 to 4, **characterized in that** said determination uses a feed-back loop automated system based on a mathematical algorithm and/or an artificial intelligence module implemented by a control module (13).

6. The method according to claims 1 to 5, wherein said method further comprises: measuring and/or identifying, by at least one other sensor (6) measuring before or/and after the biological entity(es), at least one physicochemical parameter of said liquid cell culture medium (2), said at least one physicochemical parameter being chosen from:
- temperature,
- metabolite(s),
- nutrient(s),
- protein(s)
- nucleic acid(s).

7. The method according to claims 1 to 6, **characterized in that** it further comprises a control of a temperature by injecting a thermalization liquid.

8. The method according to claims 1 to 7, **characterized in that** it further comprises a successive displacement by the liquid controller of said volume of liquid cell culture medium (VL) from said at least one closed chamber to at least one other closed chamber, said closed chambers (14, 15) being mounted in series and/or in parallel.

9. The method according to claim 1 to 8, **characterized in that** said at least one gas-permeable membrane (4) is made of a polymer with a thickness lower than 500µm and with bulk density lower than 1g/cm³ or any other material with specific gas permeabilities higher than 10 Barrer.

10. The method according to claim 9, **characterized in that** said polymer is selected from the group comprising: polymethylpentene (PMP), poly(1-trimethylsilyl-1-propyne) (PTMSP), polymethylated polymers, 2,2-bistrifluoromethyl-4,5-difluoro-1,3-dioxole (PDD) and tetrafluoroethylene (TFE) copolymers.

11. A cell culture system **characterized in that** it implements the method according to claims 1 to 10, **and in that** it comprises:
- A microfluidic device (A, D), a fluidic device (A) or fluidic assembly (A), called device (A, D), said device (A, D) comprising:
• at least two microfluidic circuits each comprising at least one microfluidic channel, one of said at least two microfluidic circuits being able to contain a flowing gas, or gas mixture (1), or sequence of gases injected by a gas controller, and at least one other of said at least two microfluidic circuits being able to contain a flowing liquid, or sequence or different liquid cell culture medium injected by a liquid controller,
• at least one gas-permeable membrane (4, 22) being able to produce a diffusion and pressure driven exchange of gas, or gas mixtures (1), between a liquid cell culture medium (2) circulating in one of the at least two microfluidic circuit and a gas, or gas mixture (1) circulating in the other of the at least two microfluidic circuit, said at least two microfluidic circuits overlapping with each other *via* the at least one gas-permeable membrane (4, 22) connecting at least one microfluidic channel of each at least two microfluidic circuits to each other,
• at least one dissolved gas(es) concentration and/or pH sensor (5) placed in the microfluidic circuit in which the liquid cell culture medium (2) is circulating and configured to collect measures of a dissolved gas(es) concentration and/or pH of said liquid cell culture medium (2);
• at least one closed chamber (14, 15);
- at least one gas controller configured to inject said gas, or gas mixture (1) into said device (A, D);
- at least one liquid controller configured to inject said liquid cell culture medium (2) into said closed chamber of said device (A, D);
- said at least one gas controller and at least one liquid controller being configured to adjust an injection flow rate and/or pressure of said gas, or gas mixture (1), or a gas mixture composition, and/or an injection flow rate and/or pressure of said liquid cell culture medium (2), based on said measured dissolved gas(es) concentration and/or pH of said liquid cell culture medium (2), fluid flow rates to be less or equal to 50ml/min, local pressures to be less or equal to 100 bar and a predetermined dissolved gas, or gas mixture (1), composition and/or pH of the liquid cell culture medium (2).

12. A cell culture system according to claim 11, **characterized in that** when said cell culture system (S) comprises a fluidic assembly (A), then said cell culture system (S) further comprises:
- a multi-well cell culture plate (20) comprising a plurality of wells,
- said fluidic assembly (A) comprising a microfluidic device (D) mounted on said multi-well cell culture plate (20),
and said microfluidic device (D) further comprises:
- a fluids-routing layer (25) comprising at least one connection orifice or at least one fluidic connection duct (251) allowing the attachment of at least one fluid connector coming from said at least one gas controller and/or said at least one liquid controller,
- a manifold or liquid-routing layer (21) forming the base of the microfluidic device (D), said manifold (21) comprising at least two nozzles or orifices or ducts (214), and at least one cavity (215), said at least one cavity (215) forming with at least one well of said multi-well cell culture plate (20) said at least one closed chamber (14, 15) containing a volume of liquid cell culture medium (VL), and said at least two nozzles or orifices or ducts (214) forming at least one liquid inlet (IN) and at least one liquid outlet (OUT) connecting the volume of liquid cell culture medium (VL) contained in the at least one closed chamber (14, 15) to an exterior of said at least one closed chamber (14, 15),
- said at least two microfluidic circuits each comprising at least one microfluidic channel (211, 235), said at least two microfluidic circuit extending from at least one connection orifice or at least one fluidic connection duct (251, 255) of said fluids-routing layer (25) to a fluid outlet (10) of said microfluidic device (D) or to said at least two nozzles or orifices or ducts (215) of said manifold (21), *via* said at least one microfluidic channel (211), one of said at least two microfluidic circuits being able to contain the flowing gas, or gas mixture (1) continuously injected by said gas controller, and at least one other of said at least two microfluidic circuits being able to contain said flowing liquid cell culture medium continuously injected by said liquid controller,
- said at least two microfluidic circuits overlapping with each other *via* the at least one gas-permeable membrane (4, 22) connecting at least one microfluidic channel (235, 211) of each at least two microfluidic circuits to each other,
- the at least one dissolved gas(es) concentration and/or pH sensors (3, 5) are embedded inside said at least one microfluidic channel (211) of the at least one of the at least two microfluidic circuits in which the liquid cell culture medium is flowing after or before and after the gas-permeable membrane (4, 22).

13. A cell culture system according to claims 11 to 12, **characterized in that** said at least one dissolved gas(es) concentration and/or pH sensor(s) (5) collects measures of the dissolved gas(es) concentration and/or pH of said liquid cell culture medium (2) after the production of the diffusion and pressure driven exchange of gas, or gas mixtures (1), between said liquid cell culture medium (2) and the gas, or gas mixture (1) through said at least one gas-permeable membrane (4, 22).

14. A cell culture system according to claim 13, **characterized in that** said device (A, D) further comprises at least one dissolved gas(es) concentration and/or pH sensor (3) configured to collect measurements of dissolved gas(es) concentration and/or pH of said liquid cell culture medium before the production of the diffusion and pressure driven exchange of gas, or gas mixtures (1), between said liquid cell culture medium (2) and the gas, or gas mixture (1) through said at least one gas-permeable membrane (4, 22).

15. A cell culture system according to claim 13 or 14, **characterized in that** said device (A, D) further comprises at least one dissolved gas(es) concentration and/or pH sensor (12) configured to collect measurements of dissolved gas(es) concentration and/or pH of said liquid cell culture medium after said liquid cell culture medium has passed through at least one biological entity (7) culture in the at least one closed chamber (15).

16. A cell culture system according to claims 11 to 15, **characterized in that** it further comprises a control module (13) configured to display on a human-machine interface the data collected by the at least one dissolved gas(es) concentration and/or pH sensor(s) (5, 3).

17. A cell culture system according to claims 11 to 15, **characterized in that** it further comprises a control module (13) configured to:
- determine the injection flow rate and/or pressure of said gas, or gas mixture (1) and/or the injection flow rate and/or pressure of said liquid cell culture medium (2) based on said dissolved gas(es) concentration and/or pH of said liquid cell culture medium measurements, fluid flow rates to be less or equal to 50ml/min, local pressures to be less or equal to 100 bar and predetermined dissolved gas, or gas mixture (1), composition and/or pH of the liquid cell culture medium (2), using a feed-back loop automated system based on a mathematical algorithm and/or an artificial intelligence module implement by the control module;
- transmit a command to adjust the injection flow rate and/or pressure of said gas, or gas mixture (1) to the gas controller and/or the injection flow rate and/or pressure of said liquid cell culture medium (2) to the liquid controller.

18. A cell culture system according to claims 11 to 17, **characterized in that** said device (A, D) comprises at least one other sensor (6) being able to measure and/or identify at least one physicochemical parameter of said liquid cell culture medium (2), said at least one physicochemical parameter being chosen from:
- temperature,
- metabolite(s),
- nutrient(s),
- protein(s),
- nucleotide acid(s).

19. A cell culture system according to claims 11 to 18, **characterized in that** said device (A, D) comprises at least one other parallel microfluidic circuit, having at least one microfluidic channel in which an injected thermalization liquid flows into.

20. A cell culture system according to claim 11 to 19, **characterized in that** said at least one microfluidic circuit containing the flowing liquid cell culture medium (2) fluidically connects the volume of liquid cell culture medium (VL) contained in said at least one closed chamber (14) to another volume of liquid cell culture medium contained in at least one other closed chamber (15), said volume of liquid cell culture medium (2) being successively displaced by said at least one liquid controller from said at least one closed chamber (14) to said at least one other closed chamber through said at least one microfluidic circuits.
